# EUROPEAN PATENT SPECIFICATION

(11) **EP 1 802 251 B1**
(45) Date of publication and mention of the grant of the patent: **24.10.2018**
(21) Application number: 05857308.0
(22) Date of filing: 30.09.2005
(51) Int. Cl.: A61F 2/00, D04B 21/12, D04B 21/16

(54) **SURGICAL IMPLANT**
CHIRURGISCHES IMPLANTAT
IMPLANT CHIRURGICAL

(30) Priority: 21.10.2004 DE 102004051487
(43) Date of publication of application: 04.07.2007
(73) Proprietor: Johnson & Johnson Medical GmbH, 22851 Norderstedt (DE)
(72) Inventor: WALTHER, Christoph, 24568 Kattendorf (DE); SCHULDT-HEMPE, Barbara, 24576 Bad Bramstedt (DE); WOHLERT, Stephen, 22848 Norderstedt (DE)
(74) Representative: Uexküll & Stolberg
(86) International application number: PCT/EP2005/010614
(87) International publication number: WO 2006/092159

(56) References cited:
- EP-A- 0 898 944
- WO-A-01/80773
- GB-A- 2 222 954
- US-A- 4 796 603
- US-A- 5 743 917

## Description

The invention relates to a surgical implant which is suitable in particular for closing and covering soft-tissue defects in mammals.

Surgical implants for repairing soft-tissue defects, e.g. hernias, are known which have a three-dimensional area extending beyond an areal mesh structure. For example, EP 0 621 014 B1 reveals two parallel mesh sections which, in their middle area, are held at a distance by a formed-loop spacer knit. An implant similar in terms of its geometric structure is disclosed in WO 2004/017869 A1. In EP 0 999 805 B1, a three-dimensional formed-loop spacer knit is described in which channels are formed between two opposite porous sides. WO 01/80773 A1 discloses an implant which has an areal base structure and whose middle area is designed as a collapsible projection.

Such implants are sometimes awkward to use and do not give the desired treatment success.

EP 0 898 944 A discloses a prosthetic device to repair a hernia, which comprises a canal insert made of sheet material for extending through the hernia. The canal insert is attached at one end to a collar and at the other end to a base that is to be placed on the inside of the hernial cavity. The base can be designed as a pouch which can be filled, via the canal, with fiber or foam material so as to constitute a ball of fiber or foam in the pouch to absorb abdominal pressure elastically while spreading the force exerted at the hernial canal over a portion of the wall of the hernial cavity surrounding the canal.

The object of the invention is to make available a surgical implant which is suitable for repairing soft-tissue defects, is easy to handle and gives good medical results.

This object is achieved by a surgical implant having the features of Claim 1. Advantageous embodiments of the invention are set out in the dependent claims.

The surgical implant according to the invention has an areal base structure and at least one projection which is absorbable or partially absorbable. The projection is designed to take up at least half the implant's own weight of body fluids (e.g. serum).

The concept of the implant according to the invention permits good handling because the projection can be inserted into the defect to be treated, while the areal base structure rests on the adjacent body tissue. The areal base structure can readily grow in and, in so doing, secures the projection which, over the course of time, is absorbed or partially absorbed. Before its absorption, the projection stabilizes the defect, particularly if it has sufficient compressibility to adapt to anatomical conditions. The projection is also open-pored and designed to take up body fluids, so that undesired body fluids are withdrawn from the area surrounding the defect. This leads, for example, to possible seromas or accumulations of fluid being limited to the area of the projection and to fibrin deposition in this area with optimized wound healing; haemostatic effects are also obtained. Depending on the choice of material, the implant according to the invention can have anti-adhesive properties in its entirety or in partial areas.

The projection is preferably designed for insertion into an abdominal wall defect (e.g. the orifice of a hernia) and preferably withstands an overpressure in the abdominal cavity of more than 90 mbar. This means that the projection retains its correct fit in the abdominal wall defect even when there is a corresponding overpressure in the abdominal cavity. Such overpressures arise, for example, during coughing, but in most cases are below 90 mbar. The projection is preferably designed to withstand an overpressure of more than 140 mbar or even of more than 200 mbar. The resistance to an overpressure can be achieved not only through the geometry, but also by the surface properties of the implant (adherence to body tissue). Additional securing of the implant can be achieved by fixing the base structure on the abdominal wall.

There are numerous possibilities for the configuration of the at least one projection of the implant according to the invention. Thus, the projection can have a maximum thickness (i.e. a thickness which is measured without the thickness of the base structure when the projection sits on the base structure) which is 0.5 to 50 times as great as the thickness of the base structure. The projection can be designed in one piece with the base structure or can be placed as a separate part onto the base structure.

In a preferred embodiment, the projection has several stages. The projection can also have a protuberance. It can also be hollow or can be filled with a filler material. In a particularly preferred embodiment, the implant has a formed-loop spacer knit, e.g. in the area of the projection (or also of several projections).

Examples of the shape of the projection are cylindrical (including staged or stepped forms), prismatic, prismatic with several stages, conical, frustoconical, mushroom-shaped or ring-shaped. A particularly advantageous shape for treatment of hernias is a champagne-cork-shape, see below. Coffee-filter-shaped configurations are also conceivable.

The base structure preferably contains a knitted mesh (e.g. a crochet galloon fabric), but other configurations (e.g. as formed-loop spacer knits, non-wovens or drawn-loop spacer knits) are also conceivable.

The fact that a formed-loop spacer knit is particularly advantageous has already been noted. A formed-loop spacer knit can in fact be produced as a three-dimensional, open-pore structure in one working step. It is thus also possible for a projection designed in one piece with the base structure to be produced as a formed-loop spacer knit. Other examples for structures of the projection are non-wovens, drawn-loop knits, other kinds of knits (also crochet galloon fabrics), but meshes are also conceivable. Moreover, the projection can be shaped by deformation of said structures in order to extend the projection away from the areal base structure or enlarge it.

In a particularly preferred embodiment, the projection has an attachment area which is sited on the base structure and which is adjoined by a cylindrical or frustoconical middle part, pointing away from the base structure, and by a widening at the end remote from the base structure. The shape of such a projection bears a similarity to that of a champagne cork. The attachment area can jut off from the middle part, e.g. in a manner similar to the brim of a hat, which makes it easier to secure the projection on the base structure. It is also conceivable, however, that the attachment area is not especially discernible in relation to the middle part. Deviations form a strictly cylindrical or frustoconical shape of the middle part are conceivable. By means of the widening at the end of the middle part, which widening, for example, is designed as a cylindrical disc, a particularly firm fit of the projection in an abdominal wall defect is achieved, even against an overpressure of about 300 mbar. In an advantageous embodiment, the attachment area and the middle part are made in one piece from a plane structure by thermal deformation; the widening can in this case be applied as a separate part to the middle part and, for example, can be sewn onto the latter. The attachment area, the middle part and/or the widening can have a spacer knit.

If the implant has several projections, at least one projection can be provided on the top side of the base structure and at least one projection can be provided on the underside of the base structure. Such a design is expedient depending on the application of the implant.

In a preferred embodiment of the invention, the projection is charged with an active substance or a medicament. An example of this is an initially hollow projection which is filled with a collagen sponge, the collagen sponge carrying the active substance. For example, the collagen sponge can be impregnated with gentamycin.

The materials used for the surgical implant according to the invention are in principle all absorbable and non-absorbable materials that are biocompatible and are customary in the prior art. While the areal base structure can be non-absorbable, partially absorbable (i.e. provided with absorbable and with non-absorbable materials) or absorbable, the at least one projection is partially absorbable or is absorbable.

Examples of non-absorbable materials are non-absorbable polymers, polyolefins, polypropylene, fluorine-containing polyolefins (e.g. a mixture of polyvinylidene fluoride and a copolymer of vinylidene fluoride and hexafluoropropylene sold by Ethicon under the name "Pronova"), polyester and polyamides.

Examples of absorbable materials are absorbable natural polymers (e.g. collagens), absorbable synthetic polymers, polyhydroxy acids, polylactides, polyglycolides, polycaprolactones, polydioxanones and copolymers and mixtures of such substances.

Particularly suitable absorbable materials are a copolymer sold by Ethicon under the tradename "Vicryl" and composed of glycolide and lactide in the ratio of 90:10 (also called Polyglactin 910), poly-p-dioxanone (PDS), a copolymer sold by Ethicon under the tradename "Monocryl" and composed of glycolide and s-caprolactone (also called Polyglecaprone 25), a copolymer sold by Ethicon under the tradename "Panacryl" and composed of glycolide and lactide in the ratio of 5:95 (which can be provided with a coating composed of a copolymer of 90% caprolactone and 10% glycolide) and polyglycolic acid (sold by B. Braun under the tradename "Dexon").

The invention is explained in more detail below with reference to illustrative embodiments and to the drawings, in which:
- Figure 1: shows a schematic longitudinal section through an embodiment of the implant according to the invention in accordance with Example 1,
- Figure 2: shows a schematic plan view of the implant according to Figure 1,
- Figure 3: shows a pattern (with lozenge structure) for a formed-loop spacer knit,
- Figure 4: shows a pattern (with closed pore structure) for a formed-loop spacer knit,
- Figure 5: shows a perspective schematic view of a formed-loop spacer knit with closed structure,
- Figure 6: shows a perspective schematic view of a further embodiment of a formed-loop spacer knit with closed structure,
- Figure 7: shows a schematic longitudinal section through an embodiment of the implant according to the invention in accordance with Example 2, and
- Figure 8: shows a schematic longitudinal section through an embodiment of the implant according to the invention in accordance with Example 3.

### Example 1

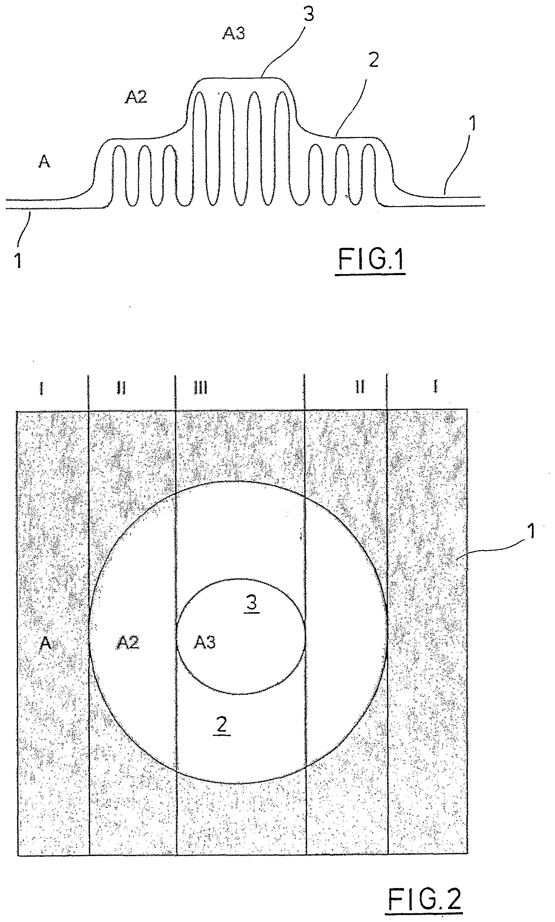

Figure 1 shows a schematic longitudinal section, not true to scale, through an embodiment of a surgical implant with a two-stage projection. Figure 2 shows the same embodiment in a schematic plan view.

This implant has an areal base structure 1, a projection 2, and a projection 3 whose height (thickness), related to the plane defined by the top side of the base structure 1, is greater than that of the projection 2. In this illustrative embodiment, the base structure 1 and the two projections 2 and 3 are made in one piece from a formed-loop spacer knit, as will be explained in more detail below. The surfaces defining the base structure 1 and the projections 2 and 3 in the plan view are designated by A, A2 and A3, see Figure 1 and Figure 2.

The formed-loop spacer knits used in this example and also in the following examples were produced on a double-bar Raschel knitting machine RD6N from the company Mayer (double-section knitting machine with three to six bars). It is possible to pattern different base structures (of a formed-loop spacer knit, not to be confused with the areal base structure 1 of the implant) on the technical upper side and underside, for which purpose two guide bars (bars 1 + 2 / bars 5 + 6) can be used in each case. The pile connection between the base structures is produced with the inner bars (bars 3 + 4). It is not absolutely essential for all six bars to work at the same time, but, in order to produce a formed-loop spacer knit, at least three bars are needed. Either one bar each for the two base structures and for the pile, or, in the production of a formed-loop spacer knit with one-sided pile/plush, two bars are used for the base structure and one for the pile, since otherwise there is insufficient stitch-pile connection.

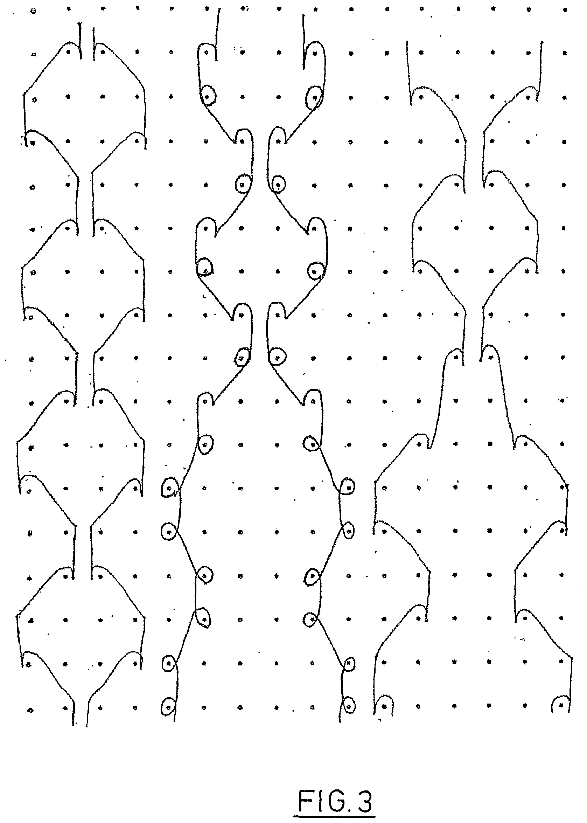

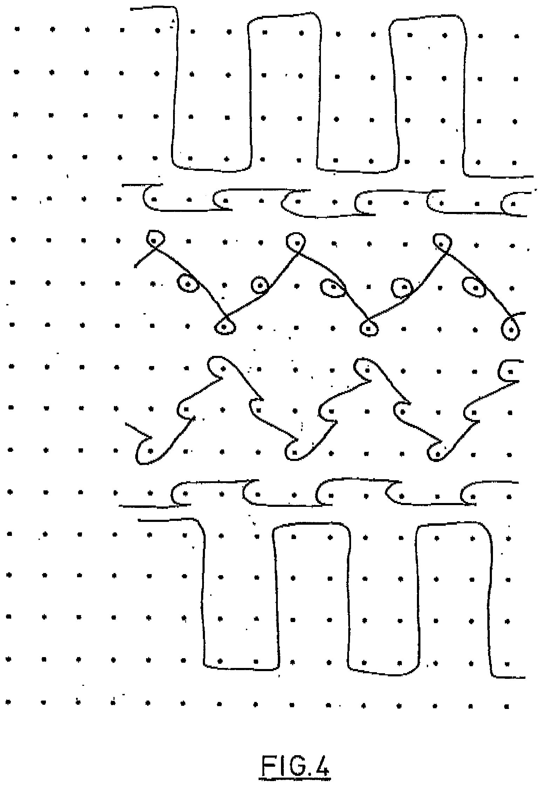

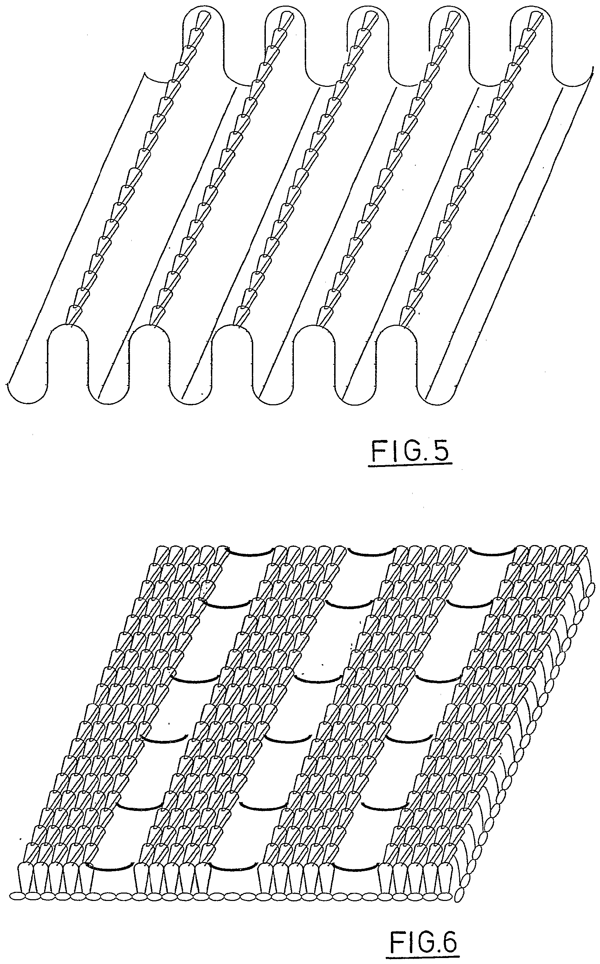

Figure 3 shows an embodiment for a formed-loop spacer knit with lozenge structure in a pattern familiar to the skilled person (pattern I). Figure 4 shows a pattern of a formed-loop spacer knit with closed pore structure (pattern II). Schematic, perspective views of examples of formed-loop spacer knits with closed pore structure are shown in Figures 5 and 6.

In pattern I (Figure 3), a lozenge structure can be seen on both sides, with closed or open pores depending on the needle position. Such patterns can be worked with three to six needle bars. Moreover, the mass per unit area of the formed-loop spacer knit can be controlled by drawing all the needles in or by omitting individual needles. By this process, the mass per unit area can be reduced or increased by up to 50%.

Pattern II (Figure 4) has a closed pore structure on both sides. Here too, the pattern can be modified by the position of the needles or the drawing-in of the needles. With partial drawing-in or even complete omission of the outer bar, a furrow-like structure is produced in the longitudinal direction. Depending on the repeat, strips with a width starting from 0.3 mm can be produced. Partial height differences in the mesh connection are possible through different thread drawin and through modification of the pattern warp. A particularly suitable material is a combination of absorbable material (in the base structure of the formed-loop spacer knit) and of non-absorbable material (pile).

A pattern described in US 6,443,964 B1 with five guide bars and a sequence of open and closed meshes is produced in four rhythm. The single or double pile thread is tied off twice as a closed pillar stitch without lateral bar shift. This provides a further example of a formed-loop spacer knit with straight show-through.

For the embodiment according to Figures 1 and 2, a formed-loop spacer knit produced on a double-section knitting machine with three to six bars is formed on the surfaces A, A2 and A3, specifically:
Surface A: base structure, on one side or both sides with lozenge structure or closed pore structure (e.g. as in above patterns I and II),
Surface A2: base structure plus additional incorporation of a pile, racking of the pile rail over two needles,
Surface A3: base structure plus additional incorporation of one or two piles, racking of the pile rails over four needles.

In Figure 2, the zones of different thread systems are designated by I, II and III. Depending on the base structure and pile selection, the systems are full or in repeat, in the example:
Thread system I: 1 full/ 1 empty
Thread system II: full
Thread system III: full

The implant according to Figures 1 and 2 is composed of a combination of absorbable and non-absorbable thread material. Non-absorbable material is used for surface A (base structure 1 of the implant), and, for surfaces A2 and A3 (projections 2 and 3), 50% absorbable and 50% non-absorbable material is used, or 70% absorbable and 30% non-absorbable material. The percentages are in % by weight. An example of a suitable absorbable material is "Monocryl" (see above), and a suitable non-absorbable material is polypropylene. Numerous other details on the choice of material are provided above and also in the following examples.

Further examples for formed-loop spacer knits and similar material are:
- formed-loop spacer knits which, towards the outside (towards the abdominal cavity), include a polypropylene mesh and, directed into the interior of a projection, comprise "Monocryl" material;
- formed-loop spacer knits based on a polypropylene mesh, a spacer of "Monocryl" and a "Monocryl" mesh;
- drawn-loop spacer knits;
- knits with nonwovens (by the so-called Malimo method);
- formed-loop spacer knits produced on a Raschel knitting machine with four bars;
- formed-loop spacer knits produced on a double-section crochet galloon machine with four to six bars.

In connection with the surgical implant, formed-loop spacer knits are suitable as material for the base structure and the at least one projection of the implant on account of a number of advantageous properties: formed-loop spacer knits initially permit a relatively large mass (e.g. of the projection) together with adequate stiffness and compressibility upon introduction, e.g. into a hernial orifice, can have large pores, do not protrude sharply into the abdominal cavity when suitably designed, and adapt to the anatomical conditions. Moreover, formed-loop spacer knits with an absorbable part have, particularly in the moist environment of the body, good adhesion to tissue, in contrast for example to meshes made of pure polypropylene, and this fact also leads to good incorporation of the implant.

### Example 2

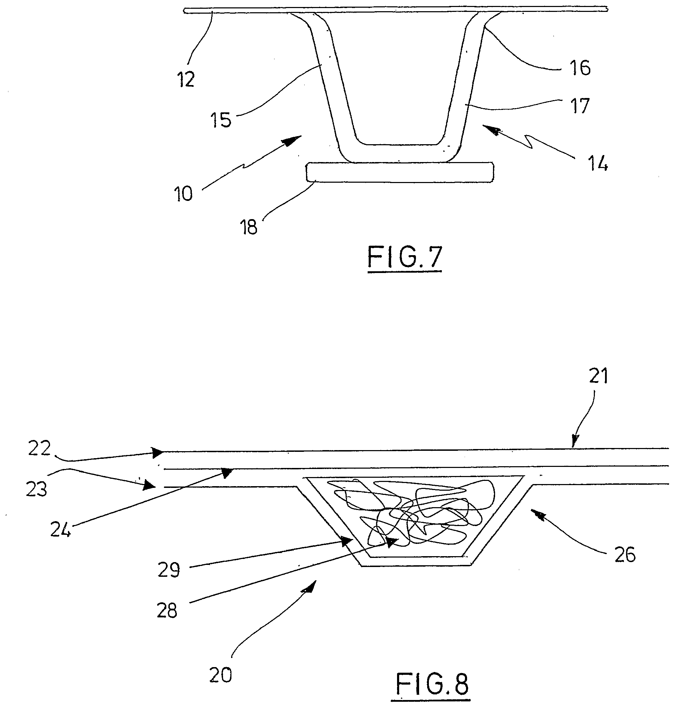

Figure 7 is a schematic view (not true to scale) of a further embodiment of the surgical implant. In this example, the areal base structure of the implant, designated here by 10, is designed as a flexible mesh 12 to which a champagne-cork-shaped projection 14 made of a formed-loop spacer knit is attached. The projection 14 has two parts, namely an integrally connected part 15, which is made up of an attachment area 16 shaped like the brim of a hat and bearing on the mesh 12, and of a frustoconical middle part 17, and also, secured separately, a widening 18. The widening 18 is sewn securely onto the middle part 17.

In the illustrative embodiment, the mesh 12 is an "Ultrapro" mesh (trademark of Ethicon for a composite mesh made of the absorbable monofilament "Monocryl" (see above) and of the non-absorbable monofilament "Prolene" (polypropylene; see above)) in the format of ca. 90 mm x 150 mm with rounded corners.

The projection 14 in this illustrative embodiment is made up of two sections 15, 18 of a formed-loop spacer knit (each of absorbable "Monocryl" and non-absorbable "Prolene", e.g. in the manner of patterns I and II from Example 1). The part 15 is produced from a flat formed-loop spacer knit by thermal deformation at temperatures of between 90°C and 150°C and has a height of ca. 15 mm. The widening 18 has a round cross section with a diameter of ca. 25 mm to 45 mm and is punched out from the formed-loop spacer knit with an ultrasonic punching device; the edge area fuses in the punching operation. The thickness of the widening 18 in the illustrative embodiment is ca. 2 mm.

The mesh 12, the part 15 and the widening 18 are sewn together with a non-absorbable monofilament thread, for example of polypropylene. After they have been sewn together, the implant 10 is washed and sterilized using ethylene oxide.

To repair a hernia with the aid of the implant 10, the part 15 is inserted into the hernial orifice so that the widening 18, which can bend because of its flexibility through the aperture of the hernial orifice, comes to lie beneath the body tissue (fascia) containing the hernial orifice, but above the peritoneum, and the stabilizing mesh 12 comes to lie on the fascia. The widening 18 ensures a secure fit and prevents slipping of the implant 10 at an overpressure in the abdominal cavity.

To investigate the fitting properties of the implant 10, a space acted upon by compressed air was covered with a synthetic skin of 4 mm thickness and having an opening of 20 mm diameter. An implant 10 with a widening 18 of 30 mm in diameter was inserted into this opening; its frustoconical part 15 had a maximum diameter of 30 mm and a minimum diameter of 15 mm. This implant was forced out of the opening in the synthetic skin only at a pressure of greater than 300 mbar.

Variants of the implant 10 are listed here by way of example:
- The mesh 12 lies flat across the opening of the part 15, as in Figure 7.
- The mesh 12 is thermally shaped and forms a central well into which the part 15 is sewn.
- The formed-loop spacer knit is washed before assembly and thermally fixed on a frame at temperatures of between 80°C and 150°C in a dry nitrogen stream (annealing).

- The formed-loop spacer knit can be replaced by a mesh having an absorbable portion.
- The formed-loop spacer knit is replaced by a mesh of polypropylene (proportion ca. 20 g/m² to 30 g/m²) and Monocryl (proportion ca. 100 g/m² to 350 g/m²); crochet galloon or Raschel fabric.
- Shape variants of the part 15 and of the widening 18, e.g. with coffee-filter-like or ring-like shapes.
- Material variants, e.g. in the absorbable part poly-p-dioxanone, "Vicryl" (see above) and in the non-absorbable part "Pronova" (see above), other polyolefins, other fluorine-containing polyolefin threads.
- The part 15 is provided with a filler (see also Example 3), e.g. a composite of "Monocryl" (see above) and poly-p-dioxanone. In this case, for example, "Monocryl" monofilament (5 mil (0.127 mm) diameter) and poly-p-dioxanone yarn (multifilament yarn 5 x 12 dpf) are entwined, the twisted yarn is then knitted on a circular knitting machine and, with unravelling, knitted on a flat knitting machine to give a knitted shawl and the latter shrunk at ca. 120°C to a frustoconical shape and sewn into the interior of the part 15.
- The part 15 and/or the widening 18 are filled with a collagen fabric which, if appropriate, is charged with one or more active substances (see as Example 3).

### Example 3

Figure 8 shows a schematic longitudinal section through a further embodiment of a surgical implant 20 with a filler.

The base structure 21 of the implant 20 is formed by two layers 22, 23 of a partially absorbable knit and, between these, an absorbable foil 24 (as adhesion means). For the layers 22, 23, suitable materials are, for example, "Ultrapro" meshes, but also meshes with a higher "Monocryl" content (which permits better thermoforming and can improve handling). The layer 23 is thermally deformed to a frustoconical shape, which leads to formation of a projection 26, so that part of the layer 23 also belongs to the projection 26. The projection 26 contains, as filler 28, a mixture of absorbable materials with different melt points, one component serving for connection to the base structure and another as a filler material with defined compression properties and for taking up fluid. The filler 28 is held together by a textile sleeve 29.

In the illustrative embodiment, the implant 20 is produced as follows: two absorbable materials are first entwined as two-step yarn 170S/150Z and worked on an RL circular knitting machine to give filler 28. Part of the circular knit hose obtained is unravelled and worked on an RR knitting machine, by which means the sleeve 29 is obtained. The layer 23 is then thermally deformed and, after introduction of the filled sleeve 29, bonded or connected to the sheet 22 via the foil 24.

The projection 26 is provided with the filler 28 so as to be able to take up a greater compression force and/or to release an active substance contained in the filler and/or to form a reservoir for body fluids, e.g. serum or blood or blood clots.

Examples of materials that can be used for the filler are:
- mixtures of absorbable yarns and/or monofilaments, e.g. a composite of "Monocryl" monofilaments (see above) and poly-p-dioxanone yarns; see also the variant cited in Example 2;
- collagen foam, also charged with one or more active substances, e.g. impregnated with an antibiotic (e.g. gentamycin or the commercial product "Septocoll" from Merck);
- a "Monocryl" nonwoven (circular knits, flat knits, thermal shaping/shrinking), in which case, with a mixture containing relatively little poly-p-dioxanone yarn, shrinkage takes place even at quite low temperatures.

### Example 4

The take-up of body fluids by the surgical implant, in particular the (at least one) projection, was tested using a formed-loop spacer knit of "Monocryl" monofilaments (5 mil (0.127 mm) diameter) and polypropylene monofilaments (3.5 mil (0.089 mm) diameter). To simulate the effect of the degradation of the "Monocryl" part in vitro, square specimens of the formed-loop spacer knit measuring 10 mm x 10 mm (0.03 g) were placed for 4 days, 7 days and 11 days in a buffer solution (pH 7.26) at a temperature of 40.9°C.

The specimens were then each immersed for 1 minute in a Petri dish containing coagulable sheep's blood (with 10 IU/ml Na heparin), removed, briefly allowed to drip, and then placed on a Petri dish until complete coagulation, and weighed.

The mean value for in each case 5 specimens was a net uptake of blood of 0.18 g, 0.21 g, 0.30 g and 0.25 g for fresh spacer knits and those pretreated for 4 days, 7 days or 11 days in the buffer solution. The formed-loop spacer knit can therefore bind a multiple of its own weight (0.03 g) of body fluids, and, after controlled degradation of the "Monocryl", the uptake capacity is greater than in fresh specimens.

## Claims

1. Surgical implant, with an areal base structure (1; 12; 22, 23, 24) and at least one projection (2, 3; 14; 26) which is absorbable or partially absorbable, **characterized in that** the at least one projection (2, 3; 14; 26) is designed to take up at least half the implant's own weight of body fluids.

2. Implant according to Claim 1, **characterized in that** the base structure (1; 12; 22, 23, 24) has a predetermined thickness, and the projection (2, 3; 14; 26) has a maximum thickness which is 0.5 to 50 times as great as the thickness of the base structure (1; 12; 22, 23, 24).

3. Implant according to Claim 1 or 2, **characterized in that** the projection (2, 3; 14; 26) is designed to be inserted into an abdominal wall defect and withstands an overpressure in the abdominal cavity of more than 90 mbar, preferably of more than 140 mbar, and particularly preferably of more than 200 mbar.

4. Implant according to one of Claims 1 to 3, **characterized in that** the projection (2, 3) is designed in one piece with the base structure (1).

5. Implant according to one of Claims 1 to 3, **characterized in that** the projection (14) is placed onto the base structure (12).

6. Implant according to one of Claims 1 to 5, **characterized in that** the projection (2, 3) has several stages.

7. Implant according to one of Claims 1 to 6, **characterized in that** the projection (14) has a protuberance (15).

8. Implant according to one of Claims 1 to 7, **characterized in that** the projection (14) is hollow.

9. Implant according to one of Claims 1 to 7, **characterized in that** the projection (26) is filled with a filler material (28).

10. Implant according to one of Claims 1 to 9, **characterized in that** the projection (2, 3; 14; 26) has one of the shapes chosen from the following group: cylindrical, cylindrical with several stages, prismatic, prismatic with several stages, conical, frustoconical, mushroom-shaped, ring-shaped, champagne-cork-shaped, coffee-filter-shaped.

11. Implant according to one of Claims 1 to 10, **characterized in that** the implant (1, 2, 3; 10) has a formed-loop spacer knit.

12. Implant according to one of Claims 1 to 11, **characterized in that** the base structure (1; 12; 22, 23, 24) has at least one of the structures included in the following list: meshes, formed-loop spacer knits, nonwovens, drawn-loop knits, formed-loop knits, crochet galloon.

13. Implant according to one of Claims 1 to 12, **characterized in that** the projection (2, 3; 14; 26) has at least one of the structures included in the following list: meshes, formed-loop spacer knits, nonwovens, drawn-loop knits, formed-loop knits, crochet galloon.

14. Implant according to Claim 1, **characterized in that** the projection (14) has an attachment area (16) which is sited on the base structure (12) and which is adjoined by a cylindrical or frustoconical middle part (17), pointing away from the base structure (12), followed by a widening (18) arranged at the end remote from the base structure (12).

15. Implant according to Claim 14, **characterized in that** the attachment area (16) and the middle part (17) are formed in one piece from a plane structure by thermal deformation.

16. Implant according to Claim 15, **characterized in that** the attachment area (16) and the middle part (17) and/or the widening (18) have a formed-loop spacer knit.

17. Implant according to one of Claims 1 to 13, **characterized in that** the base structure has a top side and an underside, and **in that** at least one projection is provided on the top side, and **in that** at least one projection is provided on the underside.

18. Implant according to one of Claims 1 to 17, **characterized in that** the projection (2, 3; 14; 26) is charged with an active substance.

19. Implant according to one of Claims 1 to 18, **characterized in that** the base structure (1; 12; 22, 23, 24) has at least one of the substances chosen from the following list: non-absorbable polymers, polyolefins, polypropylene, fluorine-containing polyolefins, mixtures of polyvinylidene fluoride and copolymers of vinylidene fluoride and hexafluoropropylene, polyesters, polyamides; absorbable natural polymers, collagens, absorbable synthetic polymers, polyhydroxy acids, polylactides, polyglycolides, polycaprolactones, polydioxanones, copolymers and mixtures of such substances.

20. Implant according to one of Claims 1 to 19, **characterized in that** the projection (2, 3; 14; 26) has at least one of the substances chosen from the following list: non-absorbable polymers, polyolefins, polypropylene, fluorine-containing polyolefins, mixtures of polyvinylidene fluoride and copolymers of vinylidene fluoride and hexafluoropropylene, polyesters, polyamides; absorbable natural polymers, collagens, absorbable synthetic polymers, polyhydroxy acids, polylactides, polyglycolides, polycaprolactones, polydioxanones, copolymers and mixtures of such substances.

## Patentansprüche

1. Chirurgisches Implantat, mit einer flächigen Grundstruktur (1; 12; 22, 23, 24) und mindestens einem Vorsprung (2, 3; 14; 26), der resorbierbar oder teilresorbierbar ist, **dadurch gekennzeichnet, dass** der mindestens eine Vorsprung (2, 3; 14; 26) zur Aufnahme des mindestens 0,5-fachen des Implantanteigengewichts an Körperflüssigkeiten eingerichtet ist.

2. Implantat nach Anspruch 1, **dadurch gekennzeichnet, dass** die Grundstruktur (1; 12; 22, 23, 24) eine vorgegebene Dicke hat und der Vorsprung (2, 3; 14; 26) eine maximale Dicke hat, die 0,5 bis 50 mal so groß ist wie die Dicke der Grundstruktur (1; 12; 22, 23, 24).

3. Implantat nach Anspruch 1 oder 2, **dadurch gekennzeichnet, dass** der Vorsprung (2, 3; 14; 26) zum Einsetzen in einen Bauchwanddefekt eingerichtet ist und einem Überdruck im Bauchraum von mehr als 90 mbar, vorzugsweise von mehr als 140 mbar und besonders bevorzugt von mehr als 200 mbar standhält.

4. Implantat nach einem der Ansprüche 1 bis 3, **dadurch gekennzeichnet, dass** der Vorsprung (2, 3) einstückig mit der Grundstruktur (1) ausgestaltet ist.

5. Implantat nach einem der Ansprüche 1 bis 3, **dadurch gekennzeichnet, dass** der Vorsprung (14) auf der Grundstruktur (12) aufgesetzt ist.

6. Implantat nach einem der Ansprüche 1 bis 5, **dadurch gekennzeichnet, dass** der Vorsprung (2, 3) mehrere Stufen aufweist.

7. Implantat nach einem der Ansprüche 1 bis 6, **dadurch gekennzeichnet, dass** der Vorsprung (14) eine Ausstülpung (15) aufweist.

8. Implantat nach einem der Ansprüche 1 bis 7, **dadurch gekennzeichnet, dass** der Vorsprung (14) hohl ist.

9. Implantat nach einem der Ansprüche 1 bis 7, **dadurch gekennzeichnet, dass** der Vorsprung (26) mit einem Füllmaterial (28) gefüllt ist.

10. Implantat nach einem der Ansprüche 1 bis 9, **dadurch gekennzeichnet, dass** der Vorsprung (2, 3; 14; 26) eine der aus der folgenden Gruppe ausgewählten Formen hat: zylindrisch, mehrstufig zylindrisch, prismatisch, mehrstufig prismatisch, kegelartig, kegelstumpfartig, pilzartig, ringartig, champagnerkorkenartig, kaffeefilterartig.

11. Implantat nach einem der Ansprüche 1 bis 10, **dadurch gekennzeichnet, dass** das Implantat (1, 2, 3; 10) ein Abstandsgewirke aufweist.

12. Implantat nach einem der Ansprüche 1 bis 11, **dadurch gekennzeichnet, dass** die Grundstruktur (1; 12; 22, 23, 24) mindestens eine der in der folgenden Liste enthaltenen Strukturen aufweist: Netze, Abstandsgewirke, Vliese, Gestricke, Gewirke, Häkelgalonware.

13. Implantat nach einem der Ansprüche 1 bis 12, **dadurch gekennzeichnet, dass** der Vorsprung (2, 3; 14; 26) mindestens eine der in der folgenden Liste enthaltenen Strukturen aufweist: Netze, Abstandsgewirke, Vliese, Gestricke, Gewirke, Häkelgalonware.

14. Implantat nach Anspruch 1, **dadurch gekennzeichnet, dass** der Vorsprung (14) einen an der Grundstruktur (12) gelegenen Ansatzbereich (16), daran anschließend ein von der Grundstruktur (12) weg weisendes zylinder- oder kegelstumpfartiges Mittelteil (17) und an dem der Grundstruktur (12) abgewandten Ende eine Verbreiterung (18) aufweist.

15. Implantat nach Anspruch 14, **dadurch gekennzeichnet, dass** der Ansatzbereich (16) und das Mittelteil (17) einstückig aus einer planen Struktur durch thermische Verformung ausgebildet sind.

16. Implantat nach Anspruch 15, **dadurch gekennzeichnet, dass** der Ansatzbereich (16) sowie das Mittelteil (17) und/oder die Verbreiterung (18) ein Abstandsgewirke aufweisen.

17. Implantat nach einem der Ansprüche 1 bis 13, **dadurch gekennzeichnet, dass** die Grundstruktur eine Oberseite und eine Unterseite hat und dass an der Oberseite mindestens ein Vorsprung vorgesehen ist und dass an der Unterseite mindestens ein Vorsprung vorgesehen ist.

18. Implantat nach einem der Ansprüche 1 bis 17, **dadurch gekennzeichnet, dass** der Vorsprung (2, 3; 14; 26) mit einem Wirkstoff beladen ist.

19. Implantat nach einem der Ansprüche 1 bis 18, **dadurch gekennzeichnet, dass** die Grundstruktur (1; 12; 22, 23, 24) mindestens eine der aus der folgenden Liste ausgewählten Substanzen aufweist: nichtresorbierbare Polymere, Polyolefine, Polypropylen, fluorhaltige Polyolefine, Mischungen aus Polyvinylidenfluorid und Copolymeren aus Vinylidenfluorid und Hexafluorpropen, Polyester, Polyamide; resorbierbare natürliche Polymere, Kollagene, resorbierbare synthetische Polymere, Polyhydroxysäuren, Polylactide, Polyglykolide, Polycaprolactone, Polydioxanone, Copolymere und Mischungen derartiger Substanzen.

20. Implantat nach einem der Ansprüche 1 bis 19, **dadurch gekennzeichnet, dass** der Vorsprung (2, 3; 14; 26) mindestens eine der aus der folgenden Liste ausgewählten Substanzen aufweist: nichtresorbierbare Polymere, Polyolefine, Polypropylen, fluorhaltige Polyolefine, Mischungen aus Polyvinylidenfluorid und Copolymeren aus Vinylidenfluorid und Hexafluorpropen, Polyester, Polyamide; resorbierbare natürliche Polymere, Kollagene, resorbierbare synthetische Polymere, Polyhydroxysäuren, Polylactide, Polyglykolide, Polycaprolactone, Polydioxanone, Copolymere und Mischungen derartiger Substanzen.

## Revendications

1. Implant chirurgical, avec une structure de base surfacique (1 ; 12 ; 22, 23, 24) et au moins une saillie (2, 3 ; 14 ; 26) qui est absorbante ou partiellement absorbante, **caractérisé en ce que** l'au moins une saillie (2 ; 3 ; 14 ; 26) est conçue pour recueillir au moins la moitié du poids de l'implant en liquides organiques.

2. Implant selon la revendication 1, **caractérisé en ce que** la structure de base (1 ; 12 ; 22, 23, 24) a une épaisseur prédéterminée, et la saillie (2, 3 ; 14 ; 26) a une épaisseur maximale qui est 0,5 à 50 fois plus grande que l'épaisseur de la structure de base (1 ; 12 ; 22, 23, 24).

3. Implant selon la revendication 1 ou 2, **caractérisé en ce que** la saillie (2, 3 ; 14 ; 26) est conçue pour être insérée dans un défaut de la paroi abdominale et résiste à une surpression dans la cavité abdominale de plus de 90 mbar, de préférence de plus de 140 mbar, et de manière particulièrement préférée de plus de 200 mbar.

4. Implant selon l'une quelconque des revendications 1 à 3, **caractérisé en ce que** la saillie (2, 3) est conçue en une seule pièce avec la structure de base (1).

5. Implant selon l'une quelconque des revendications 1 à 3, **caractérisé en ce que** la saillie (14) est placée sur la structure de base (12).

6. Implant selon l'une quelconque des revendications 1 à 5, **caractérisé en ce que** la saillie (2, 3) a plusieurs étages.

7. Implant selon l'une quelconque des revendications 1 à 6, **caractérisé en ce que** saillie (14) a une protubérance (15).

8. Implant selon l'une quelconque des revendications 1 à 7, **caractérisé en ce que** la saillie (14) est creuse.

9. Implant selon l'une quelconque des revendications 1 à 7, **caractérisé en ce que** la saillie (26) est remplie d'un matériau de remplissage (28).

10. Implant selon l'une quelconque des revendications 1 à 9, **caractérisé en ce que** la saillie (2, 3 ; 14 ; 26) a l'une des formes sélectionnées dans le groupe suivant : cylindrique, cylindrique à plusieurs étages, prismatique, prismatique à plusieurs étages, conique, tronconique, en forme de champignon, annulaire, en forme de bouchon de champagne, en forme de filtre à café.

11. Implant selon l'une quelconque des revendications 1 à 10, **caractérisé en ce que** l'implant (1, 2, 3 ; 10) a un tissu maille d'espacement à boucles formées.

12. Implant selon l'une quelconque des revendications 1 à 11, **caractérisé en ce que** la structure de base (1 ; 12 ; 22, 23, 24) a au moins l'une des structures incluses dans la liste suivante : treillis, tissus mailles d'espacement à boucles formées, non-tissés, tissus mailles à boucles tirées, tissus mailles à boucles formées, gallon crocheté.

13. Implant selon l'une quelconque des revendications 1 à 12, **caractérisé en ce que** la saillie (2, 3 ; 14 ; 26) a au moins l'une des structures incluses dans la liste suivante : treillis, tissus mailles d'espacement à boucles formées, non-tissés, tissus mailles à boucles tirées, tissus mailles à boucles formées, gallon crocheté.

14. Implant selon la revendication 1, **caractérisé en ce que** la saillie (14) a une surface de fixation (16) qui est située sur la structure de base (12) et qui est jointe par une partie intermédiaire cylindrique ou tronconique (17), pointant dans la direction opposée à la structure de base (12), suivie d'un élargissement (18) disposé au niveau de l'extrémité à distance de la structure de base (12).

15. Implant selon la revendication 14, **caractérisé en ce que** la surface de fixation (16) et la partie intermédiaire (17) sont formées en une seule pièce à partir d'une structure plane par déformation thermique.

16. Implant selon la revendication 15, **caractérisé en ce que** la surface de fixation (16) et la partie intermédiaire (17) et/ou l'élargissement (18) ont un tissu maille d'espacement à boucles formées.

17. Implant selon l'une quelconque des revendications 1 à 13, **caractérisé en ce que** la structure de base a un côté supérieur et un côté inférieur, et **en ce qu'**au moins une saillie est fournie sur le côté supérieur, et **en ce qu'**au moins une saillie est fournie sur le côté inférieur.

18. Implant selon l'une quelconque des revendications 1 à 17, **caractérisé en ce que** la saillie (2, 3 ; 14 ; 26) est chargée d'une substance active.

19. Implant selon l'une quelconque des revendications 1 à 18, **caractérisé en ce que** la structure de base (1 ; 12 ; 22, 23, 24) a au moins l'une des substances sélectionnées dans la liste suivante : polymères non absorbants, polyoléfines, polypropylène, polyoléfines contenant du fluor, mélanges de fluorures de polyvinylidène et de copolymères de fluorure de vinylidène et d'hexafluoropropylène, polyesters, polyamides ; polymères naturels absorbants, collagènes, polymères synthétiques absorbants, polyhydroxy acides, polylactides, polyglycolides, polycaprolactones, polydioxanones, copolymères et mélanges de ces substances.

20. Implant selon l'une quelconque des revendications 1 à 19, **caractérisé en ce que** la saillie (2, 3 ; 14 ; 26) a au moins l'une des substances sélectionnées dans la liste suivante : polymères non absorbants, polyoléfines, polypropylène, polyoléfines contenant du fluor, mélanges de fluorures de polyvinylidène et de copolymères de fluorure de vinylidène et d'hexafluoropropylène, polyesters, polyamides ; polymères naturels absorbants, collagènes, polymères synthétiques absorbants, polyhydroxy acides, polylactides, polyglycolides, polycaprolactones, polydioxanones, copolymères et mélanges de ces substances.
